# EUROPÄISCHE PATENTANMELDUNG

(11) **EP 0 841 332 A1**
(43) Veröffentlichungstag der Anmeldung: **13.05.1998**
(21) Anmeldenummer: 97118602.8
(22) Anmeldetag: 25.10.1997
(51) Int. Cl.: C07D 307/46, C07D 307/32, C07C 69/145, C07C 47/263

(54) **Verfahren zur Herstellung von Alpha-Methylen-Beta-Methyl-Gamma-Butyrolacton und als Zwischenprodukt wertvolle 4-Hydroxy-Butyraldehyde sowie 2-Hydroxy-3-Methylen-4-Methyltetrahydrofuran**

(30) Priorität: 07.11.1996 DE 19645766
(71) Anmelder: Degussa Aktiengesellschaft, 60311 Frankfurt (DE)
(72) Erfinder: Höpp, Mathias Dr., D-63599 Biebergemünd-Kassel (DE); Hahm, Torsten Dr., D-63456 Hanau-Kleinauheim (DE); Köhler, Klaus, D-63512 Klein-Krotzenburg (DE); Hasskerl, Thomas Dr., D-61476 Kronberg (DE)

(57) **Zusammenfassung**

Eine von Isobuten ausgehende Pd-katalysierte Acetoxylierung mit anschließender Hydroformylierung liefert Aldehyde, welche in α-Stellung mittels Mannich-Reaktion zu den entsprechenden α-Methylen-substituierten Aldehyden umgesetzt werden können.

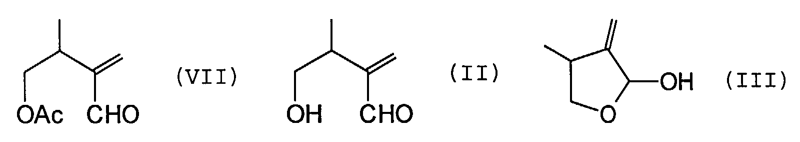

Für die Verbindungen (VII/II/III) wird Substanzschutz beantragt, da sie wertvolle Intermediate zur Synthese von α-Methylen-β-methyl-γ-butyrolacton darstellen. (VII/II/III) können leicht durch Oxidation und Ringschluß, im Falle von (VII) durch Umsetzung, zu dem Zielprodukt umgewandelt werden.

Die bisher nicht in befriedigenden Ausbeuten oder nur mittels teurer Chemikalien oder schwierig im technischen Maßstab einzusetzender Apparaturen herstellbare Zielverbindung kann mit dem neuen Verfahren sehr vorteilhaft großtechnisch dargestellt werden.

Copolymer zur Herstellung wärmeformbeständiger Formmassen.

## Beschreibung

Die Erfindung betrifft ein neuartiges Verfahren zur Herstellung von α-Methylen-β-methylbutyrolacton. Genanntes Lacton kann vorteilhafterweise zur Herstellung wärmeformbeständiger Formmassen durch Copolymerisation mit geeigneten Monomeren, wie Alkylmethacrylaten oder Styrol, verwendet werden. Die Erfindung bezieht sich auch auf neue Zwischenprodukte, nämlich 2-Methylen-3-methyl-4-hydroxybutyraldehyd (II), 2-Hydroxy-4-methyltetrahydrofuran sowie 2-Methylen-3-methyl-4-acetoxybutyraldehyd (VII).

Eine Möglichkeit zur Herstellung von α-Methylen-β-alkylbutyrolactonen wird in Chem. Express, 7(12), 901-4 (1992) beschrieben. Zunächst werden durch UV-Bestrahlung von Alkylidenmalonsäure in Alkoholen als Lösungsmitteln die entsprechenden α-Carboxy-β-alkylbutyrolactone hergestellt. Anschließende Mannich-Reaktion liefert die α-Methylen-β-alkylbutyrolactone.

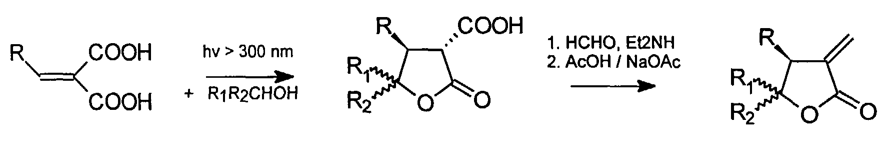

In Liebigs Ann. Chem. (3), 177-81 (1992) wird die Umsetzung von Aldehyden mit Ph₃P:C(COOEt)CH₂COOH zu RCH:C(COOEt)CH₂COOH beschrieben. Durch Behandlung dieser Verbindung mit Natriumdiethyldihydridoaluminat erhält man die entsprechenden β-Alkylidenbutyrolactone. Hydrierung der Doppelbindung liefert die β-Alkylbutyrolactone, die anschließend in einer Mannich-Reaktion in die α-Methylen-β-alkylbutyrolactone überführt werden.

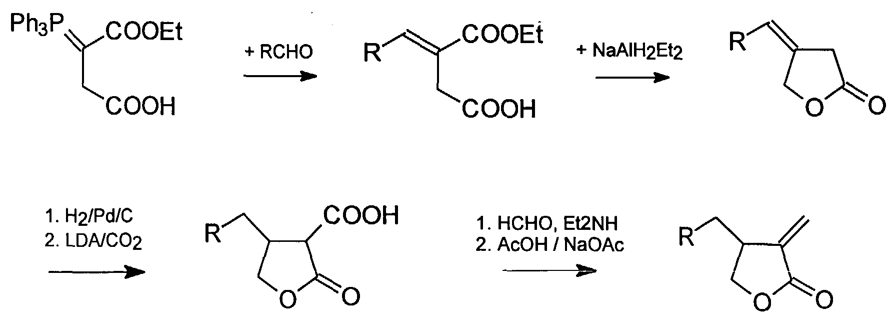

Ein einfacherer Zugang zu den β-Alkylbutyrolactonen ausgehend von einem Aldehyd und Glyoxylsäure wird in J. Org. Chem. 46, 4889 (1981) und J. Med. Chem. 31, 893 (1988) beschrieben. Zunächst erhält man ein 4-Alkyl-5-hydroxy-2(5H)-furanon, welches durch Reduktion in das β-Alkylbutyrolacton überführt wird.

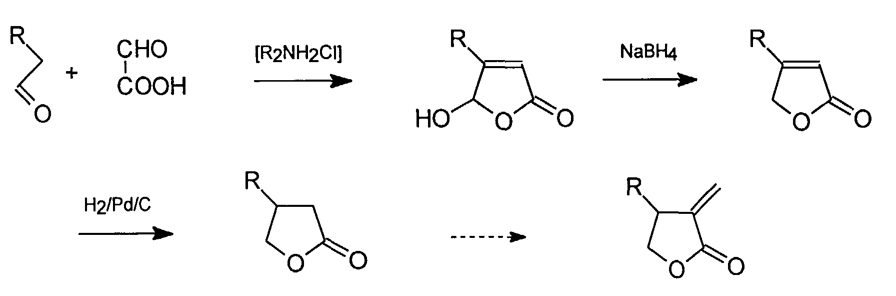

Für die Überführung der Butyrolactone in die α-Methylenbutyrolactone werden in der Literatur viele Verfahren beschrieben, die bereits in Reviews zusammengefaßt wurden (Synth. Commun. 5, 245 (1975), Synthesis 1975, 67, Heterocycles 24, 441 (1986), Synthesis 1986, 157). Allen diesen Verfahren ist es eigen, reine Laborverfahren zu sein, die nur unter Verwendung relativ teurer Chemikalien möglich sind. Ebenso sind viele der erwähnten Reaktionsschritte im technischen Maßstab nicht durchführbar oder erfordern den Einsatz von kostspieligen technischen Einrichtungsgegenständen. Ein Grund dafür mag in der geringen Acidität des α-H-Atoms der Esterfunktion, welches zunächst aktiviert werden muß, liegen.

Will man die üblichen und schwierig durchzuführenden Deprotonierungs- oder Enolisierungs- und Alkylierungsschritte umgehen, so ist ein Beispiel für eine Aktivierung des α-H-Atoms die Umsetzung des Butyrolactons mit Oxalsäurediethylester zu dem α-Ethyloxalylbutyrolacton. Dieses kann anschließend mit Formaldehyd umgesetzt werden J. Org. Chem. 42(7), 1180 (1977). Eine Vereinfachung zu einer Eintopfsynthese unter Weglassen der Zwischenisolierung des α-Ethyloxalylbutyrolactons wird in Macromolecules 12, 546 (1979) beschrieben.

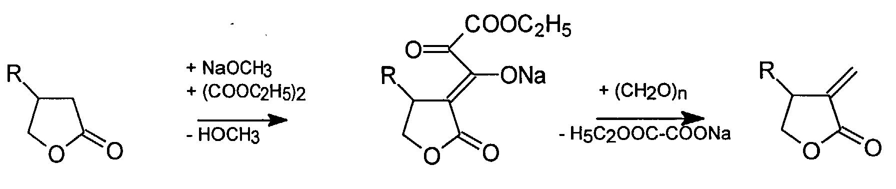

Die Darstellung von enantomerenreinem α-Methylen-β-methylbutyrolacton wird in J. Med. Chem. 30, 1948-1951 (1987) beschrieben. 4(R)-α-Methylen-β-methylbutyrolacton wird ausgehend von dem entsprechenden enantiomerenreinen Methyl-β-hydroxyisobutyrat hergestellt, 4(S)-α-Methylen-β-methylbutyrolacton ausgehend von kommerziell erhältlichem 4(S)-β-Methylbutyrolacton. In beiden Fällen wird in der letzten Stufe die Methylengruppe unter Verwendung von Eschenmosers Salz H₂C:NMe₂⁺I⁻ eingeführt.
Für alle bisher veröffentlichten Herstellprozesse gilt, daß diese nicht ohne weiteres in den kommerziellen Maßstab übertragbar sind und die Verwendung teurer Chemikalien sowie teure oder nur schwierig durchführbare verfahrenstechnische Schritte beinhalten.

Aufgabe der Erfindung ist es deshalb ein Verfahren zur Herstellung von α-Methylen-β-methylbutyrolacton zur Verfügung zu stellen, welches vor allem unter dem Gesichtspunkt, diese Verbindungen mit verbesserten Ausbeuten und preiswerteren Ausgangsmaterialien in gängigen großtechnischen Anlagen in kommerziell interessantem Maßstab verfügbar zu machen, zu sehen ist.
Diese und nicht näher genannte Aufgaben werden durch ein Verfahren der eingangs erwähnten Art gelöst, das die Merkmale des kennzeichnenden Teils des Anspruchs 1 aufweist. Vorteilhafte Verfahrensmodifikationen sind Gegenstand der auf Anspruch 1 rückbezogenen abhängigen Unteransprüche.

Dadurch, daß man 2-Methylen-3-methyl-4-hydroxybutyraldehyd (II) und/oder das tautomere 2-Hydroxy-3-methylen-4-methyltetrahydrofuran (III) in Gegenwart eines Oxidationsmittels oxidiert und anschließend zum Lacton (I) zyklisiert, erhält man dieses in vorteilhafter Weise und in hohen Ausbeuten.

Ebenso vorteilhaft kann die 2-Methylen-3-methyl-4-acetoxybuttersäure (IV) zum Lacton (I) umgeestert werden. Die Säure (IV) erhält man in besonders vorteilhafter Weise durch Reaktion von 2-Methylen-3-methyl-4-acetoxybutyraldehyd (VII) mit einem Oxidationsmittel.

Man behandelt α,β-ungesättigte Aldehyde der Formeln (II) und/oder (III) sowie (VII) erst mit einem Oxidationsmittel und estert das Produkt (IV) anschließend zum Lacton (I) um.

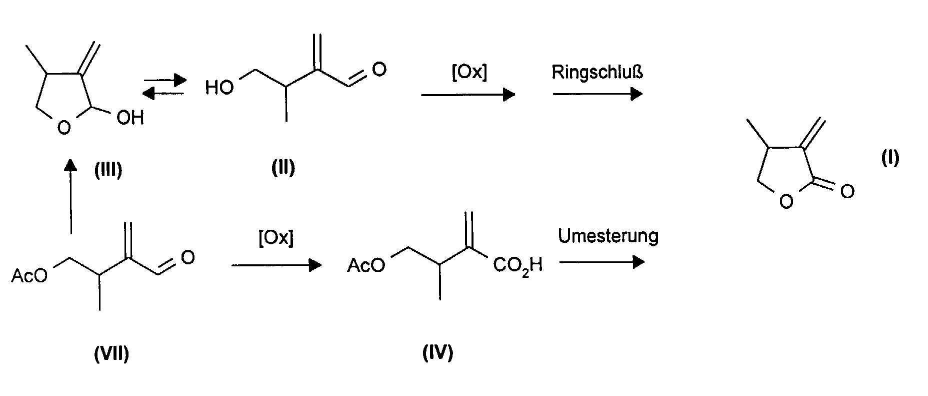

So erhält man in vorteilhafter Weise mit sehr hohen Ausbeuten das gewünschte Produkt (I).

Zur Oxidation von α,β-ungesättigten Aldehyden der Formel (II) werden in der Literatur die verschiedensten Methoden beschrieben, welche ebenfalls bei der Herstellung von α-Methylen-β-methylbutyrolacton (I) anwendbar sind. Die Oxidation kann sowohl in der Flüssigphase als auch in der Gasphase durchgeführt werden (Houben-Weyl, Bd IV, 1a, Seite 136-137, Georg Thieme Verlag Stuttgart 1981, Houben-Weyl, Bd IV, 1a, Seite 247-250, Georg Thieme Verlag Stuttgart 1981, Houben-Weyl, Bd VIII, Seite 407-413, Georg Thieme Verlag Stuttgart 1952).
Mögliche Verfahren in der Flüssigphase werden unter Tetrahedron 37, 2091 (1981) vorgestellt. Die dort angeführten Verfahren wie Oxidation mit Siberoxid, Caro'scher Säure, Mangandioxid und Natriumchlorit sind alle anwendbar zur Herstellung von α-Methylen-β-methylbutyrolacton.
Bei der Oxidation von 2-Methylen-3-methyl-4-hydroxybutyraldehyd (II/III) entsteht in äußerst vorteilhafter Weise direkt das gewünschte α-Methylen-β-methylbutyrolacton (I), während bei der Oxidation von 2-Methylen-3-methyl-4-acetoxybutyraldehyd (VII) noch eine Umesterung erfolgen muß. Erfolgt die Oxidation in einem sauren Medium, so erfolgt auch in diesem Fall direkt die Cyclisierung, wie z. B. bei der Oxidation mit Ammoniumperoxodisulfat in 85%iger Schwefelsäure (J. Org. Chem. 33, 2525 (1968)). Hierbei entsteht in beiden Fällen direkt das gewünschte α-Methylen-β-methylbutyrolacton (I). Es wurde nun gefunden, daß auch die Oxidation mit Persäuren wie z. B. Gleichgewichtsperessigsäure (40%ige Peressigsäure) das gewünschte Produkt (I) liefert. Als Nebenprodukte werden zwar die entsprechenden Epoxide gebildet, jedoch verläuft die Oxidation der Aldehydfunktion so schnell, daß das Hauptprodukt im Falle von 2-Methylen-3-methyl-4-hydroxybutyraldehyd (II/III) das α-Methylen-β-methylbutyrolacton (I) ist. Bei der Oxidation von 2-Methylen-3-methyl-4-acetoxybutyraldehyd (VII) entsteht ein Gemisch aus 2-Methylen-3-methyl-4-acetoxybuttersäure (IV) und α-Methylen-β-methylbutyrolacton (I). Verbindung (IV) muß zur Vervollständigung der Reaktion noch weiter umgeestert werden.
Eine weitere Möglichkeit, α,β-ungesättigte Aldehyde wie II/III/VII zu oxidieren, besteht im Einsatz von Natriumchlorit (Acta Chem. Scand. 27, 888 (1973)). Die Umsetzung erfolgt vorzugsweise in tert. Butanol als Lösungsmittel mit Isoamylen als Chlorfänger. Im Falle des 2-Methylen-3-methyl-4-acetoxybutyraldehyd (VII) muß das Produktgemisch anschließend noch durch saure Umesterung in das α-Methylen-β-methylbutyrolacton (I) überführt werden. Für die Oxidation in der Gasphase geeignet sind prinzipiell alle Katalysatoren, wie sie auch zur Herstellung von (Meth)acrylsäure aus (Meth)acrolein verwendet werden.
In einer vorteilhaften Verfahrensmodifikation ist es möglich, 2-Methylen-3-methyl-4-acetoxybutyraldehyd (VII) zunächst zu 2-Methylen-3-methyl-4-hydroxybutyraldehyd (II/III) zu verseifen und anschließend zu oxidieren.

Die Mannich-Reaktion des 3-Methyl-4-acetoxybutyraldehyds der Formel (VIII) mit Formaldehyd kann analog zu bekannten Vorschriften zur Herstellung von α-Alkylacroleinen durchgeführt werden.

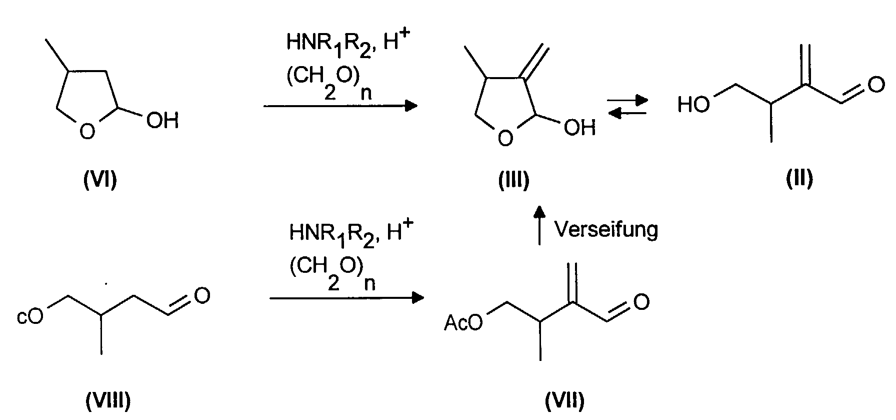

Geeignet ist sowohl die Umsetzung mit sekundären Aminen und anorganischen oder organischen Säuren (EP 58 927 (BASF AG, 1982)) als Katalysator, oder auch die Verwendung katalytischer Mengen eines Enamins (DE 28 55 505 (Ruhrchemie AG, 1978)).
3-Methyl-4-acetoxybutyraldehyd (VIII) läßt sich vorteilhaft mit Formaldehyd in Gegenwart eines sekundären Amins und einer Säure zu 2-Methylen-3-methyl-4-acetoxybutyraldehyd (VII) umsetzen. Das Molverhältnis 3-Methyl-4-acetoxybutyraldehyd (VIII) zu Formaldehyd liegt bei 0,7-2,0 : 1 und vorteilhafterweise bei 0,9-1,3 : 1. Das Verhältnis 3-Methyl-4-acetoxybutyraldehyd (VIII) zu Amin (V) liegt bei 1:1 bis 10:1. Das Verhältnis Amin (V) zu Säure liegt bei 1-2 Äquivalente Säure pro Äquivalent Amin. Der pH-Wert während der Reaktion liegt bei 2,5-7 und vorteilhafterweise bei 3-6,5. Der Formaldehyd kann sowohl als wäßrige Formalinlösung als auch als Paraformaldehyd eingesetzt werden. Es können sek. Amine der Formel (V) 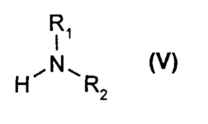 eingesetzt werden, in denen
R₁,R₂ unabhängig voneinander gleich oder verschieden, (C₁-C₁₃)-Alkyl linear oder verzweigt, Cycloalkyl, Aryl, Alkylaryl oder Aralkyl, oder zu einem gesättigten oder teilweise oder ganz ungesättigten Ring der Größe 5 oder 6 geschlossen, enthaltend 0, 1 oder 2 Heteroatome wie N,O,S oder P, sein können.
Als Säuren können alle organischen oder anorganischen Säuren, deren pKₛ-Wert kleiner als 5 ist, eingesetzt werden.
Es gilt festzustellen, daß die einsetzbaren sek. Amine und Säuren nicht auf die oben aufgelisteten Beispiele beschränkt sind.
Bei Verwendung von Dibutylamin und Schwefelsäure als Katalysator beispielsweise konnte der 2-Methylen-3-methyl-4-acetoxybutyraldehyd (VII) mit einer Ausbeute >88% hergestellt werden.

Für die Mannich-Reaktion des 4-Methyl-2-hydroxytetrahydrofuran (VI) mit Formaldehyd gelten dieselben Bedingungen mit Ausnahme des pH-Wertes. Es wurde gefunden, daß sowohl das 4-Methyl-2-hydroxytetrahydrofuran (VI) als auch das Produkt 2-Methylen-3-methyl-4-hydroxybutyraldehyd (II) gegen Säure empfindlich ist und zu höhermolekularen Folgeprodukten reagieren. Um dies während der Mannich-Reaktion zu vermeiden, ist es notwendig, den pH-Wert während der Reaktion im Bereich 6,5-7,0 zu halten. In diesem Fall gelingt die Umsetzung mit Ausbeuten von 60-80 %.
Die zweite Möglichkeit zur Herstellung des α,β-ungesättigten Aldehyds (II/III bzw. VII) besteht in der bereits in der Literatur beschriebenen Verwendung katalytischer Mengen eines Enamins (DE 28 55 505 (Ruhrchemie AG, 1978)).
Das Enamin (XI) wird dabei vorteilhafterweise zunächst durch Umsetzung eines sekundären Amins (V) mit dem Ausgangsaldehyd (VI oder VIII) gebildet und anschließend in katalytischen Mengen zur Reaktion des Formaldehyd mit dem Aldehyd (VI oder VIII) zugesetzt.

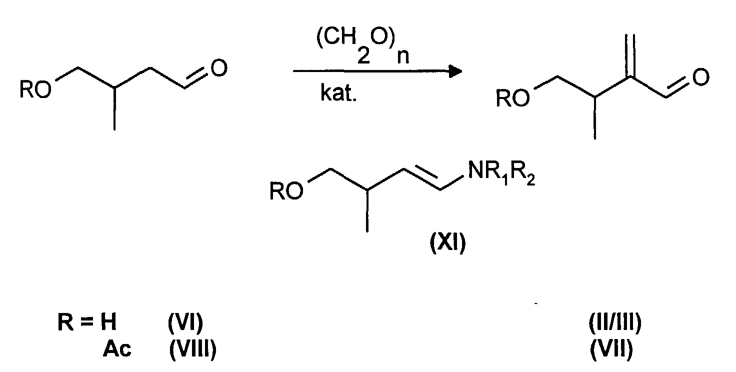

Das Amin (V) kann das oben beschriebene Substitutionsmuster aufweisen.
Ausgangsaldehyd (VI oder VIII) und Formaldehyd werden im Molverhältnis 1 : 0,7-2,0 und vorteilhafterweise im Molverhältnis 1 : 0,9-1,3 eingesetzt. Der Formaldehyd kann sowohl als wäßrige Formalinlösung als auch als Paraformaldehyd eingesetzt werden. Das Amin (V) wird mit 0,01 - 0,1 Mol je Mol Aldehyd (VI oder VIII) eingesetzt. Die Reaktion wird bei Temperaturen von 60-120 °C, vorzugsweise bei 80-100 °C durchgeführt. Die Anwesenheit eines Lösungsmittels wie Wasser oder ein Alkohol ist zweckmäßig aber nicht notwendig.
Eine ebenfalls vorteilhafte Verfahrensmodifikation ist die Umsetzung des Aldehyds (VIII) zum 2-Hydroxy-4-methyl-tetrahydrofuran (VI) durch Verseifung, bevor die oben beschriebene Mannich-Reaktion erfolgt.

Zur Herstellung der Aldehyde (VI oder VIII) wird vorzugsweise Isobutenol (X) bzw. Isobutenylacetat der Formel (IX) hydroformyliert. Die Hydroformylierung von ethylenisch ungesättigten Verbindungen mit Wasserstoff und Kohlenmonoxid als ein industriell anwendbarer Syntheseschritt zur Herstellung von Aldehyden ist bekannt und ist auch bereits für substituierte Allylalkohole in Chemiker-Ztg. 101, 343 (1977) beschrieben.
In Gegenwart eines Katalysatorsystems, bestehend aus einem Gruppe 8-10-Metall und einem Phosphinliganden bzw. Phosphitliganden, erhält man die entsprechenden Aldehyde (VI oder VIII) mit Ausbeuten von >90 %. Die Reaktion läßt sich sowohl in gängigen Lösungsmitteln als auch direkt in Substanz durchführen, wobei die besten Ausbeuten direkt in Substanz erzielt werden.
Geeignete Gruppe 8-10-Metalle sind Kobalt, Ruthenium, Rhodium, Palladium, Platin, Osmium und Iridium. Geeignete Gruppe 8-10-Metallverbindungen sind Hydride, Halogenide, organische Säuresalze, anorganische Säuresalze, Oxide, Carbonylverbindungen und Aminverbindungen dieser Metalle. Beispiele für diese Verbindungen sind die Rutheniumverbindungen Ru₃(CO)₁₂, Ru(NO₃)₃, RuCl(PPh₃)₃ und Ru(acac)₃, die Palladiumverbindungen PdCl₂, Pd(OAc)₂, Pd(acac)₂, PdCl₂(COD), Pd(PPh₃)₄, PdCl₂(PPh₃)₂, PdCl₂(CH₃CN)₂ und PdCl₂(PhCN)₂, die Osmiumverbindungen Os₃(CO)₁₂ und OsCl₃, die Iridiumverbindungen Ir₄(CO)₁₂ und IrSO₄, die Platinverbindungen K₂PtCl₄, PtCl₂(PhCN)₂, Na₂PtCl₆xH₂O, PtCl₂ und PtCl₄, die Kobaltverbindungen CoCl₂, Co(NO₃)₂, Co(OAc)₂, Co₂(CO)₈ und Co(acac)₄ und die Rhodiumverbindungen RhCl₃, Rh(NO₃)₃, Rh(OAc)₃, Rh₂O₃, Rh(acac)(CO)₂, [Rh(OAc)(COD)]₂, Rh₄(CO)₁₂, Rh₆(CO)₁₆, HRh(CO)(PPh₃)₃, [Rh(OAc)(CO)₂]₂ und [RhCl(COD)]₂ (acac ist Acetylacetonat, Ac ist eine Acetylgruppe, COD ist 1,5-Cyclooctadien und Ph ist eine Phenylgruppe). Es gilt festzustellen, daß die einsetzbaren Gruppe 8-10-Metailverbindungen nicht auf die oben aufgelisteten Beispiele beschränkt sind.
Geeignete Liganden sind sowohl Triarylphosphine als auch Triarylphosphite. Bevorzugt eingesetzt werden Bis(diarylphosphino)alkane wie Ar₂P-(CH₂)ₙ-PAr₂ mit n = 2-6. Die Arylsubstituenten können mit jedweder Gruppe substituiert sein, solange der Substituent die Hydroformylierung nicht stört. Besonders bevorzugte Beispiele für Triarylphosphine sind Triphenylphosphin, Triphenylphosphintrisulfonat, Triphenylphosphinmonosulfonat, Tris(2-methylphenyl)phosphin, Tris(2,6-dimethylphenyl)phosphin, Tris(2-isopropylphenyl)phosphin, Tris(2-phenylphenyl)phosphin, Tris(2-t-butylphenyl)phosphin, Tris(2,4-di-t-butylphenyl)phosphin und Tris(2-methyl-4-chlorphenyl)phosphin. Besonders bevorzugte Beispiele für Triarylphosphite sind Triphenylphosphit, Tris(2-methylphenyl)phosphit, Tris(2,6-dimethylphenyl)phosphit, Tris(2-isopropylphenyl)phosphit, Tris(2-phenylphenyl)phosphit, Tris(2-t-butylphenyl)phosphit, Tris(2,4-di-t-butylphenyl)phosphit und Tris(2-methyl-4-chlorphenyl)phosphit. Es gilt festzustellen, daß die einsetzbaren Liganden nicht auf die oben aufgelisteten Beispiele beschränkt sind.
Die vorteilhaften Reaktionsbedingungen sind wie folgt. Die Metallkonzentration im Reaktionsgemisch liegt zwischen 10 und 10.000 ppm und vorzugsweise zwischen 100 und 1000 ppm. Das Molverhältnis Ligand zu Metall liegt im Bereich 0,5 bis 100 und vorzugsweise im Bereich 1 bis 20. Die Temperatur für die Reaktion liegt im Bereich 0-200 °C und vorzugsweise im Bereich 50-150 °C. Der Druck liegt im Bereich 1-30 bar und vorzugsweise im Bereich 3-15 bar. Das Molverhältnis Wasserstoff zu Kohlenmonoxid liegt zwischen 10:1 und 1:10 und vorzugsweise im Bereich 1:1 bis 6:1. Die Reaktion kann sowohl ohne Lösungsmittel als auch mit Lösungsmitteln durchgeführt werden. Bei Verwendung eines Lösungsmittels sind gesättigte und aromatische Kohlenwasserstoffe geeignet, z. B. Benzol, Toluol oder Cyclohexan, Methylcyclohexan, Hexan oder Ether, z. B. Tetrahydrofuran oder Dimethoxyethan oder Ketone, z. B. Cyclohexanon, Methylisobutylketon oder Aceton. Weiterhin können Ester zum Einsatz kommen, wie z. B. Essigester oder Acetessigester.
Bei der Hydroformylierung von Isobutenylacetat (IX) entsteht als Produkt 4-Acetoxy-3-methylbutyraldehyd (VIII), bei Isobutenol (X) das entsprechende Halbacetal 4-Methyl-2-hydroxytetrahydrofuran (VI).

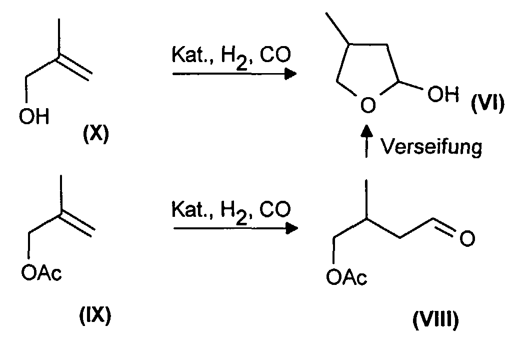

In einer weiteren bevorzugten Verfahrensmodifikation kann der 4-Acetoxy-3-methylbutyraldehyd (VIII) vor dessen weiterer Verwendung durch Verseifung in das 2-Hydroxy-4-methyl-tetrahydrofuran (VI) überführt werden.

Die gefundene, neue Synthese für α-Methylen-β-methylbutyrolacton (I) geht von Isobutenylacetat (IX) bzw. Isobutenol (X) aus, welches nach bekannten Vorschriften aus Isobuten herstellbar ist. Großtechnisch am interessantesten ist die allylische Acetoxylierung von Isobuten an modifizierten Pd-Kontakten, wie sie in verschiedenen Patenten beschrieben wird (DE 28 11 211 (Höchst AG), DE 25 06 141 (Höchst AG, 1978), DE 19 33 537 (Bayer AG, 1969)),

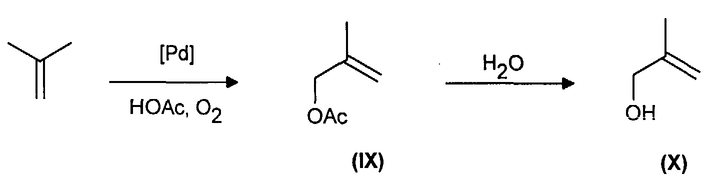

Durch anschließende Verseifung läßt sich aus (IX) Isobutenol (X) herstellen.

Gegenstand der Erfindung sind auch die in der Synthese auftretenden Verbindungen 2-Methylen-3-methyl-4-hydroxybutyraldehyd (II) sowie sein Tautomeres das 2-Hydroxy-4-methyltetrahydrofuran (III) und der 2-Methylen-3-methyl-4-acetoxybutyraldehyd (VII). Es handelt sich um neue und wertvolle Intermediate, die für die vorgestellte Synthese Schlüsselverbindungen darstellen.

Zusammenfassend kann man sagen, daß diese vorgestellte Synthese in ihrer Form die erste ist, welche in einen technischen Maßstab ohne den Einsatz teurer Chemikalien sowie ohne die Verwendung von komplizierten technischen Verfahrensapparaturen umgesetzt werden kann. Mit den Verfahren des Standes der Technik sind weder die Ausbeuten oder Reinheit noch die Kosten für das Endprodukt zu erreichen, wie sie durch das erfindungsgemäße Verfahren vorgegeben werden.

Nachfolgende Beispiele sollen zur Erläuterung der Erfindung dienen:

### Beispiel 1, Hydroformylierung von Isobutenol

216 g Isobutenol (3,0 Mol) werden mit 0,46 g HRh(CO)(PPh₃)₃ (0,5 mmol) versetzt. Als Co-Katalysator werden 1,31 g Triphenylphosphin (5,0 mmol) zugesetzt. Bei 110 °C und 10 bar Druck wird mit einem CO/H₂-Gemisch (molar 1:1) hydroformyliert, bis keine weitere Gasaufnahme mehr erfolgt.
Das Reaktionsgemisch wird von dem Katalysator abkondensiert und anschließend destilliert. Man erhält 257 g eines cis/trans-Gemisches von β-Methylbutyrolactol (84 % Ausbeute).

### Beispiel 2, Hydroformylierung von Isobutenylacetat

114 g Isobutenylacetat (1,0 Mol) werden in 300 ml Toluol gelöst und mit 0,92 g HRh(CO)(PPh₃)₃ (1,0 mmol) versetzt. Als Co-Katalysator werden 2,62 g Triphenylphosphin (10,0 mmol) zugesetzt. Bei 110 °C und 10 bar Druck wird mit einem CO/H₂-Gemisch (molar 1:1) hydroformyliert, bis keine weitere Gasaufnahme mehr erfolgt. Das Reaktionsgemisch wird von dem Katalysator abkondensiert und per GC analysiert.
Das Produktgemisch enthält 75 % 4-Acetoxy-3-methylbutyraldehyd, 15 % Isovaleraldehyd und 7 % Essigsäure.

### Beispiel 3, Hydroformylierung von Isobutenylacetat

171 g Isobutenylacetat (1,5 Mol) werden mit 0,46 g HRh(CO)(PPh₃)₃ (0,5 mmol) versetzt. Als Co-Katalysator werden 1,55 g Triphenylphosphit (5,0 mmol) zugesetzt. Bei 110 °C und 10 bar Druck wird mit einem CO/H₂-Gemisch (molar 1:1) hydroformyliert, bis keine weitere Gasaufnahme mehr erfolgt. Das Reaktionsgemisch wird von dem Katalysator abkondensiert und per GC analysiert. Das Produktgemisch enthält 91 % 4-Acetoxy-3-methylbutyraldehyd, 2,5 % Isovaleraldehyd und 2,4 % nicht umgesetztes Isobutenylacetat.

### Beispiel 4, Mannich-Reaktion von β-Methylbutyrolactol mit Formaldehyd

36,5 g Diethylamin (0,50 Mol) und 0,1 g Hydrochinon werden in 40 ml Wasser vorgelegt. Durch Zugabe von 24,5 g konz. Schwefelsäure (0,25 Mol) wird genau ein pH-Wert von 7 eingestellt. 16,5 g Paraformaldehyd (0,55 Mol) werden zugegeben und das Reaktionsgemisch wird auf 65 °C erwärmt. 51 g β-Methylbutyrolactol (0,5 Mol) werden zugetropft und das Reaktionsgemisch wird noch 3 h bei 65 °C gerührt. Anschließend wird abgekühlt und das Reaktionsgemisch mit Ether extrahiert. Die Etherphase wird am Rotationsverdampfer eingeengt. Man erhält 32 g Rohprodukt mit einer GC-Reinheit von 91 % (51 % Ausbeute).

### Beispiel 5, Herstellung von 2-Methylen-3-methyl-4-acetoxybutyraldehyd

1 g Hydrochinon, 14 g Dibutylamin (0,108 Mol) und 46 g 2N Schwefelsäure (0,092 Mol) werden in 243 g 37%iger Formaldehydlösung (3 Mol) gelöst. Unter Kühlung werden bei einer Innentemperatur von 20 °C 288 g 3-Methyl-4-acetoxybutyraldehyd (2,0 Mol) innerhalb von 40 Min. zugegeben. Es wird noch 20 Min. bei 20 °C und anschließend 1,5 h bei 70 °C gerührt. Hierbei färbt sich die zunächst klare Lösung rot und es scheidet sich eine zweite Phase ab. Nach dem Abkühlen wird die organische Phase abgetrennt und die wäßrige zweimal mit je 200 ml MTBE extrahiert. Die vereinigten org. Phasen werden über Natriumsulfat getrocknet. Nach Destillation erhält man 270,6 g 2-Methylen-3-methyl-4-acetoxybutyraldehyd (85,2 % Ausbeute).

### Beispiel 6, Oxidation von 2-Methylen-3-methyl-4-hydroxybutyraldehyd mit Gleichgewichtsperessigsäure

5,7 g 2-Methylen-3-methyl-4-hydroxybutyraldehyd (0,05 Mol) und 0,1 g BHT werden vorgelegt und auf 50 °C erwärmt. 11,4 g 40%ige Gleichgewichtsperessigsäure werden langsam zugetropft. Nach beendeter Zugabe wird eine Probe per GC analysiert. Es erfolgte quantitativer Umsatz, wobei das Hauptprodukt mit >80% das α-Methylen-β-methyl-γ-butyrolacton ist.

### Beispiel 7, Oxidation von 2-Methylen-3-methyl-4-acetoxybutyraldehyd mit Caro'scher Säure

79 g 2-Methylen-3-methyl-4-acetoxybutyraldehyd (0,5 Mol) werden in 600 ml Methanol vorgelegt. Zur Herstellung der Caro*'*schen Säure werden 114 g Ammoniumperoxodisulfat (0,5 Mol) bei 15 °C in 144 g 85%iger Schwefelsäure suspendiert. Diese Suspension wird über den Zeitraum von 1 h bei 15 °C portionsweise zu dem Reaktionsgemisch zugegeben. Es wird noch 3 h bei 15 °C nachgerührt, anschließend über Nacht bei Raumtemperatur stehen gelassen.
Das Reaktionsgemisch wird mit 600 ml Wasser versetzt und im Perforator mit Ether extrahiert.
Die Etherphase wird mit 50 g ges. Fe(II)-sulfat-Lösung gewaschen, über Natriumsulfat getrocknet und eingeengt. Man erhält 44,3 g Rohprodukt mit einem Gehalt von 83 % α-Methylen-β-methylbutyrolacton (66 % Ausbeute).

### Beispiel 8, Oxidation von 2-Methylen-3-methyl-4-acetoxybutyraldehyd mit Natriumchlorit

23,4 g 2-Methylen-3-methyl-4-acetoxybutyraldehyd (0,15 Mol), 0,25 g BHT und 21 g Isoamylen (0,3 Mol) werden in 250 ml tert. Butanol vorgelegt. Über 25 Minuten wird eine Lösung von 34 g NaClO₂ (80%ig, 0,3 Mol) und 41,4 g Natriumdihydrogenphosphatmonohydrat (0,3 Mol) in 150 ml Wasser bei 25 °C unter Kühlung zugetropft. Es wird 20 h bei Raumtemperatur nachgerührt.
Die beiden Phasen werden getrennt und die wäßrige wird zweimal mit je 250 ml Ether extrahiert. Die vereinigten org. Phasen werden dreimal mit je 100 ml Wasser gewaschen und über Natriumsulfat getrocknet. Nach Abdestillieren des Lösungsmittels verbleiben 26 g Rohprodukt mit einem Gehalt von 66 % 2-Methylen-3-methyl-4-acetoxybuttersäure (0,10 Mol)und 13 % α-Methylen-β-methylbutyrolacton (0,03 Mol). In Summe entspricht dies einer Ausbeute von 86,7 %.

### Beispiel 9, Oxidation von 2-Methylen-3-methyl-4-acetoxybutyraldehyd mit Gleichgewichtsperessigsäure

14,4 g 2-Methylen-3-methyl-4-acetoxybutyraldehyd (0,1 Mol) und 0,1 g BHT werden vorgelegt und auf 50 °C erwärmt. 23 g 40%ige Gleichgewichtsperessigsäure werden langsam zugetropft. Nach beendeter Zugabe wird noch 6 h nachgerührt und eine Probe per GC analysiert. Die Ausbeute beträgt 19 % 2-Methylen-3-methyl-4-acetoxybuttersäure und 56 % α-Methylen-β-methylbutyrolacton.

## Patentansprüche

1. Verfahren zur Herstellung von α-Methylen-β-methyl-γ-butyrolacton der Formel (I) 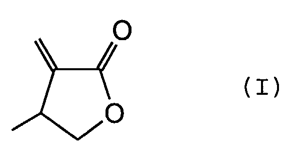 **dadurch gekennzeichnet**,
daß α-Methylen-3-methyl-4-hydroxybutyraldehyd der Formel (II) 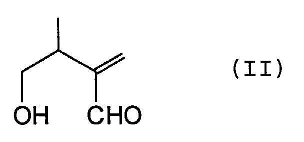 und/oder das tautomere 2-Hydroxy-3-methylen-4-methyltetrahydrofuran der Formel (III) 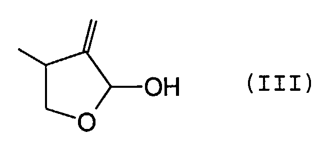 in Gegenwart eines Oxidationsmittels oxidiert und anschließend zum Lacton (I) zyklisiert werden.

2. Verfahren zur Herstellung von α-Methylen-β-methyl-γ-butyrolacton der Formel (I) 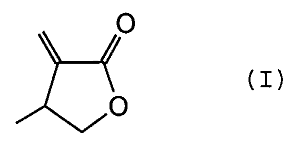 **dadurch gekennzeichnet,**
daß 2-Methylen-3-methyl-4-acetoxybuttersäure der Formel (IV) 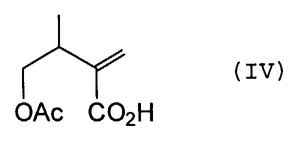 zum Lacton der Formel (I) unter Ringbildung umgeestert wird.

3. Verfahren nach Anspruch 2,
**dadurch gekennzeichnet**,
daß man 2-Methylen-3-methyl-4-acetoxybuttersäure der Formel (IV) 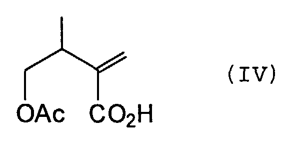 durch Reaktion von 2-Methylen-3-methyl-4-acetoxybutyraldehyd der Formel (VII) 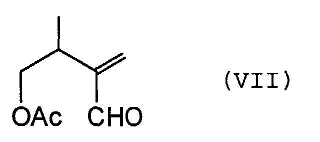 mit Oxidationsmitteln gewinnen kann.

4. Verfahren nach Anspruch 1,
**dadurch gekennzeichnet**,
daß man die Verbindungen der Formeln (II/III) 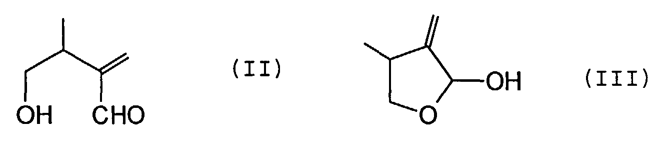 durch Reaktion von Formaldehyd oder eine Formaldehyd erzeugende Vorstufe, einer Säure und einem sekundären Amin der Formel (V) 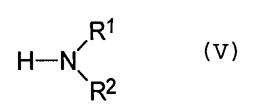 in der
R¹, R² unabhängig voneinander (C₁-C₁₃)-Alkyl, linear oder verzweigt, Cycloalkyl, Aryl oder Aralkyl sowie Alkylaryl oder zu einem gesättigten oder teilweise oder ganz ungesättigten Ring der Größe 5 oder 6 geschlossen, enthaltend 0, 1 oder 2 Heteroatome, wie S, P, N oder O, sein können,
wobei das Molverhältnis (II/III) zu Formaldehyd wie 0,7 - 2 : 1 sein kann, bevorzugt ist ein Verhältnis von 0,9 - 1,3 : 1, und das Verhältnis (II/III) zu Amin der Formel (V) von 1 : 1 bis 10 : 1 variieren, sowie das Verhältnis Amin der Formel (V) zu Säure im Bereich von 1 : 1 bis 1 : 2 liegen kann,
mit einer Verbindung der Formel (VI) 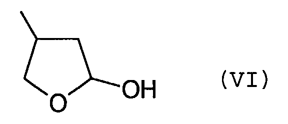 herstellen kann.

5. Verfahren nach Anspruch 1,
**dadurch gekennzeichnet**,
daß man die Verbindungen der Formeln (II/III) 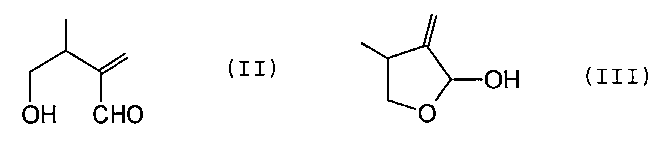 durch Verseifen der Verbindung der Formel (VII) 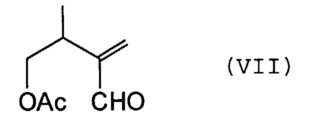 erhalten kann.

6. Verfahren nach Anspruch 3 und 5,
**dadurch gekennzeichnet**,
daß man 3-Methyl-4-acetoxybutyraldehyd der Formel (VII) 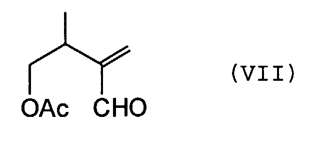 durch Reaktion von Formaldehyd oder ein Formaldehyd erzeugende Vorstufe, einer Säure und einem sekundären Amin der Formel (V) 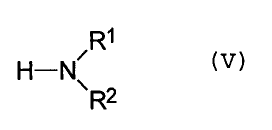 in der
R¹, R² unabhängig voneinander (C₁-C₁₃)-Alkyl, linear oder verzweigt, Cycloalkyl, Aryl oder Aralkyl sowie Alkylaryl oder zu einem gesättigten oder teilweise oder ganz ungesättigten Ring der Größe 5 oder 6 geschlossen, enthaltend 0, 1 oder 2 Heteroatome, wie S, P, N oder O, sein können,
wobei das Molverhältnis (II/III) zu Formaldehyd wie 0,7 - 2 : 1 sein kann, bevorzugt ist ein Verhältnis von 0,9 - 1,3 : 1, und das Verhältnis (II/III) zu Amin der Formel (V) von 1 : 1 bis 10 : 1 variieren, sowie das Verhältnis Amin der Formel (V) zu Säure im Bereich von 1 : 1 bis 1 : 2 liegen kann,
mit einer Verbindung der Formel (VIII) 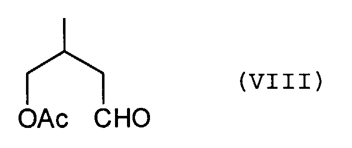 erhalten kann.

7. Verfahren nach Anspruch 4,
**dadurch gekennzeichnet**,
daß man eine Verbindung der Formel (VI) 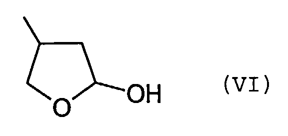 durch Hydroformylierung von Isobutenol der Formel (X) 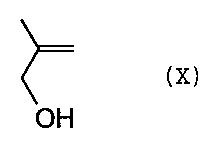 erhält.

8. Verfahren nach Anspruch 7,
**dadurch gekennzeichnet**,
daß die Hydroformylierung ausgehend von Isobutenol der Formel (X) 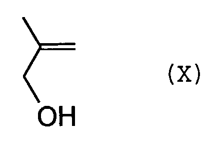 mit CO und H₂ in Gegenwart eines Katalysatorsystems, bestehend aus einem Gruppe 8 - 10-Metall und einem Phosphinliganden bzw. Phosphitliganden, ohne sowie in Gegenwart eines organischen Lösungsmittels erfolgt.

9. Verfahren nach Anspruch 4,
**dadurch gekennzeichnet**,
daß der 3-Methyl-4-acetoxybutyraldehyd der Formel (VIII) 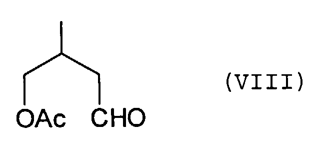 durch Verseifen in die Verbindung der Formel (VI) 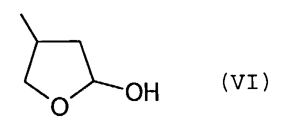 überführbar ist.

10. Verfahren nach Anspruch 6 und 9,
**dadurch gekennzeichnet**,
daß man den 3-Methyl-4-acetoxybutyraldehyd der Formel (VIII) durch Hydroformylierung von Isobutenylacetat der Formel (IX) 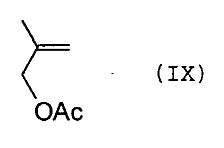 gewinnt.

11. Verfahren nach Anspruch 10,
**dadurch gekennzeichnet**,
daß die Hydroformylierung ausgehend von Isobutenylacetat der Formel (IX) 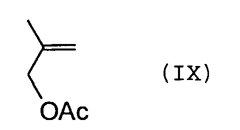 mit CO und H₂ in Gegenwart eines Katalysatorsystems, bestehend aus einem Gruppe 8 - 10-Metall und einem Phosphinliganden bzw. Phosphitliganden, ohne sowie in Gegenwart eines organischen Lösungsmittels erfolgt.

12. Verfahren nach Anspruch 8 und 11,
**dadurch gekennzeichnet**,
daß bei der Hydroformylierung die Katalysatorkonzentration im Reaktionsgemisch bei 10 - 10.000 ppm, bevorzugt sind 100 - 1.000 ppm, liegt, das Molverhältnis Ligand zu Metall einen Wert von 0,5 - 100, bevorzugt ist ein Wert im Bereich von 1 - 20, annimmt und die Temperatur zwischen 0 °C und 200 °C, vorzugsweise zwischen 50 °C und 150 °C, liegt, wobei der Druck der CO/H₂-Gasmischung 1 - 30 bar, vorzugsweise 3 - 15 bar, beträgt und das Molverhältnis H₂ zu CO zwischen den Werten 10 : 1 und 1 : 10, bevorzugt ist ein Bereich von 1 : 1 bis 6 : 1, liegt.

13. Verfahren nach Anspruch 7,
**dadurch gekennzeichnet**,
daß man Isobutenol aus Isobutenylacetat durch Verseifen herstellt.

14. Verfahren nach Anspruch 13,
**dadurch gekennzeichnet**,
daß Isobuten in Gegenwart von Palladium-Katalysatoren und Sauerstoff in Gegenwart von Eisessig allylisch acetoxyliert wird.

15. Verfahren nach Anspruch 1 und 3,
**dadurch gekennzeichnet**,
daß Verbindungen der Formeln (VIII) oder (VI) 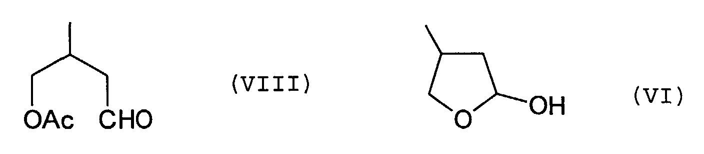 in Gegenwart von 0,7 - 2,0 Äquiv. Formaldehyd oder eine Formaldehyd erzeugende Vorstufe, bevorzugt sind 0,9 - 1,3 Äquiv., bezogen auf den eingesetzten Aldehyd (VIII oder VI), und einem Enamin (XI) 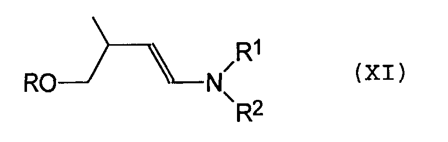 in dem
R bei Einsatz von (VIII) = Ac, bei Einsatz von (VI) = H und
R¹, R² unabhängig voneinander (C₁-C₁₃)-Alkyl, linear oder verzweigt, Cycloalkyl, Aryl oder Aralkyl sowie Alkylaryl oder zu einem gesättigten oder teilweise oder ganz ungesättigten Ring der Größe 5 oder 6 geschlossen, enthaltend 0, 1 oder 2 Heteroatome, wie S, P, N oder O, sein können,
dieses Enamin (XI) in einem Verhältnis von 0,01 - 0,1 Mol, bezogen auf den eingesetzten Aldehyd (VIII/VI), eingesetzt wird,
in einem Temperaturbereich von 60 - 120 °C, bevorzugt sind 80 - 100 °C,
zu Verbindungen der Formel (II/III) bzw. (VII) 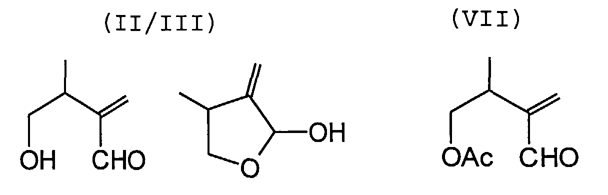 umgesetzt werden.

16. Verfahren nach Anspruch 15,
**dadurch gekennzeichnet**,
daß das Enamin der Formel (XI) aus einem Amin der Formel (V) 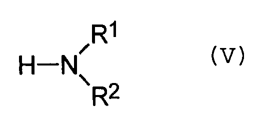 in dem
R¹, R² unabhängig voneinander (C₁-C₁₃)-Alkyl, linear oder verzweigt, Cycloalkyl, Aryl oder Aralkyl sowie Alkylaryl oder zu einem gesättigten oder teilweise oder ganz ungesättigten Ring der Größe 5 oder 6 geschlossen, enthaltend 0, 1 oder 2 Heteroatome, wie S, P, N oder O, sein können,
und dem Aldehyd der Formel (VIII) 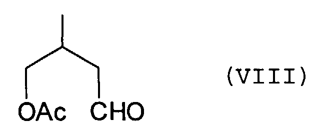 oder dem Aldehyd der Formel (VI) 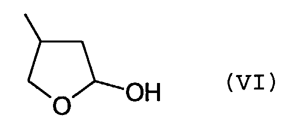 gebildet wird.

17. 2-Methylen-3-methyl-4-acetoxybutyraldehyd (VII).

18. 2-Methylen-3-methyl-4-hydroxybutyraldehyd (II).

19. 2-Hydroxy-3-methylen-4-methyltetrahydrofuran (III).
